# EUROPEAN PATENT APPLICATION

(11) **EP 3 447 142 A1**
(43) Date of publication of application: **27.02.2019**
(21) Application number: 17188028.9
(22) Date of filing: 25.08.2017
(51) Int. Cl.: C12P 5/02, C12P 3/00, C02F 11/04

(54) **IMPROVED METHOD FOR HYDROLYSIS OF BIOMASS**

(71) Applicant: Clayton Hall Farm Biogas Products Ltd., Huddersfield West Yorkshire HD8 9QE (GB)
(72) Inventor: GEMMELL, Neil, Huddersfield, West Yorkshire HD8 9QE (GB)
(74) Representative: Gill Jennings & Every LLP

(57) **Abstract**

A method for the hydrolysis of biomass in a fermenter, such as an anaerobic digester, comprising the steps of combining a microbial composition, a suitable feedstock, and a polymeric material, to form a mixture, and then digesting the mixture in the fermenter under suitable conditions to hydrolyse the biomass. The method may be for the formation of sugar, alcohol and/or carboxylic acid, such as volatile fatty acid, and preferably biogas.

## Description

### Field of the Invention

The present invention relates to an improved method for the hydrolysis of biomass in a fermenter, such as an anaerobic digester. The method is particularly useful in the production of high-value chemicals, such as biogas, sugar, alcohol and/or carboxylic acid, such as volatile fatty acid.

### Background of the Invention

Biomass is a source for many bio-based products and is one of the art. Biomass is organic material such as plant material which contain compounds such as cellulose and hemicellulose. Biomass generally comprises organic material which can be converted into fuel, heat and high-value chemicals. Microorganisms have been shown to be an environmentally friendly method to breakdown and convert biomass into useful compounds such as biogas, sugars, alcohols, and carboxylic acids.

Typically, one of the early stages of biomass conversion is the hydrolysis of complex components of biomass. Some of these components of biomass can prove difficult to breakdown, which leads to low yields and rate of formation of the end products. There is therefore a need in the industry to improve the efficiency and rate of conversion of biomass into useful products to make industrial process more economical.

One particular high-value compound that can be generated through the breakdown of biomass is biogas. The process of converting biomass to biogas is known in the art and often proceeds by anaerobic digestion. In anaerobic digestion microorganisms breakdown organic material in the absence of oxygen. Through this process biogas is released and a high nutrient digestate is also produced. The biogas produced can be used in many different applications, for instance as as a biofuel, and the high nutrient digestate can be used for instance as an effective fertiliser.

The production of biogas by anaerobic digestion occurs through a number of stages. It is understood that there are four keys stages in which biomass is converted to biogas, which are performed by microbes.

As mentioned above, the first stage is hydrolysis wherein the complex molecules that make up biomass are broken down into their constituent parts. For example, carbohydrates and fats are broken down into simple sugars and fatty acids. The next stage involves the process of acidogenesis, wherein the products of hydrolysis are converted into volatile fatty acids, alcohols, hydrogen and carbon dioxide. Some of the products formed at this stage, such as carbon dioxide, hydrogen and acetic acid can be used directly in methanogenesis. However, other compounds require further breakdown which occurs through acetogenesis. During acetogenesis the longer chain volatile fatty acids, such as propionic acid and butyric acid, are converted into carbon dioxide, hydrogen and acetic acid. The final stage of anaerobic digestion is methanogenesis. Wherein the products from acidogenesis and acetogenesis, eg. carbon dioxide, hydrogen and acetic acid, are converted into methane.

As is known, biogas primarily comprises methane but may also comprise other compounds such as carbon dioxide, and small amounts of hydrogen and hydrogen sulphide. The biogas can be purified by a number of methods which involve removing the carbon dioxide, hydrogen and hydrogen sulphide. Once the other gases have been removed or substantially reduced, the gas is referred to as biomethane. "Biomethane" is methane produced from biomass. Biomethane is often used as a biofuel. Biofuels are an attractive environmentally-friendly alternative to fossil fuels as they are produced from renewable energy sources and produce less carbon emissions.

Biogas is highly attractive as it has many uses and provides an environmentally friendly energy source. However, the yield and rate of formation of biogas by anaerobic digestion can be relatively low. Traditional methods for producing biogas use cattle slurry, but yields and rates are typically low, and therefore not economically viable. Further, on an industrial scale, biogas production can be costly and so may not be economically attractive.

The conversion of biomass into biogas and other high-value compounds relies on microorganisms to breakdown the biomass or feedstock. This process has proved difficult to optimise and efficient breakdown of the biomass or "feedstock" can be difficult to achieve. This often results in low yields of biogas and other end products. Large amounts of leftover digestate are also produced, the disposal of which can be costly. Recent improvements in the biomass processing use different combinations of microorganisms and pre-treatment of the biomass to enhance breakdown. Despite this, there is a clear and unmet need in the industry to increase yield and rate of formation of high-value compounds biomass, and in particular biogas.

### Summary of the Invention

It has been surprisingly found that it is possible to significantly improve the breakdown of biomass, and consequently the yield of biogas obtained from anaerobic digestion, by the utilisation of a polymeric material. It has been found that a typical improvement in yield of biogas may be up to approximately 35 % when a polymeric material is present during a fermentation process, such as the process of anaerobic digestion.

The presently claimed method has also demonstrated a number of other surprising benefits. For example, by incorporating the polymeric material into the anaerobic digestion the biogas is produced at a higher rate, in comparison to when no polymeric material is present. It has also been demonstrated that by using the polymeric material during anaerobic digestion more biogas is produced per gram of biomass used, which increases the efficiency of the process. The present method may require approximately 25% less biomass to produce the same amount of biogas as from anaerobic digestion in the absence of the polymeric material. When the polymeric material is present during anaerobic digestion there may also be a reduction in the amount of leftover digestate.

Without wishing to be bound by theory, it is believed that the increased yield and rate of production of biogas is achieved by a more efficient breakdown or hydrolysis of the biomass. The reduction in leftover digestate and increase in yield of the end product may indicate that a higher proportion of the biomass material is converted to the end product, and that the polymeric material improves the efficiency of the biomass hydrolysis. Therefore, the method described herein is a more efficient method for the hydrolysis of biomass.

The present invention is based at least in part on the data presented herein.

In a first aspect of the present invention there is provided a method for the hydrolysis of biomass in a fermenter, such as an anaerobic digester, comprising the steps of
(i) combining a microbial composition, a suitable feedstock, and a polymeric material, to form a mixture; and then
(ii) digesting the mixture in the fermenter under suitable conditions to hydrolyse the biomass.

In particular, a preferred feature of the first aspect of the present invention, the method is for the production of biogas in an anaerobic digester, comprising the steps of
(i) combining a microbial composition, a suitable feedstock, and a polymeric material, to form a mixture; and then
(ii) digesting the mixture in the anaerobic digester under suitable conditions to produce biogas.

A second aspect is a method for forming biomethane, comprising the steps of
a) forming biogas by the method according to the first aspect of the invention; and
b) extracting biomethane from the biogas formed in step a).

A third aspect is a kit for use in the method of producing biogas according to the first aspect of the invention, comprising a polymeric material and microbial composition suitable for use in anaerobic digestion; and instructions for its use in the method.

A fourth aspect is the use of a microbial composition and a polymeric material as defined for the first aspect of the invention, in the production of biogas by anaerobic digestion in an anaerobic digester, wherein the microbial composition is suitable for use in anaerobic digestion.

### Brief Description of the Figures

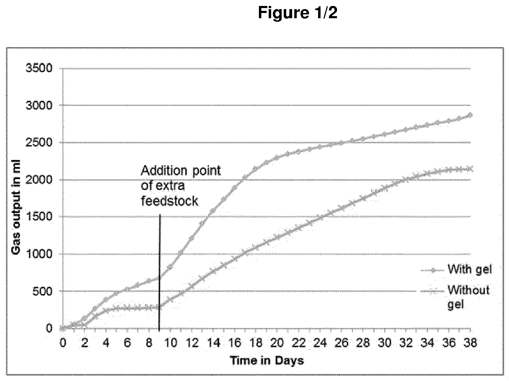

Figure 1 shows a comparison of biogas production over 38 days from a sample containing digestate and feedstock and a sample containing polymeric gel, digestate and feedstock. Feedstock was added at day 1 and a second addition of feedstock was added at day 9. The total biogas is produced at a faster rate and in a higher amount when a polymeric material ("gel") is included in the process. For instance, the amount of biogas produced after 19 days when a polymeric material is used takes around twice as long (38 days) in the absence of a polymeric material.

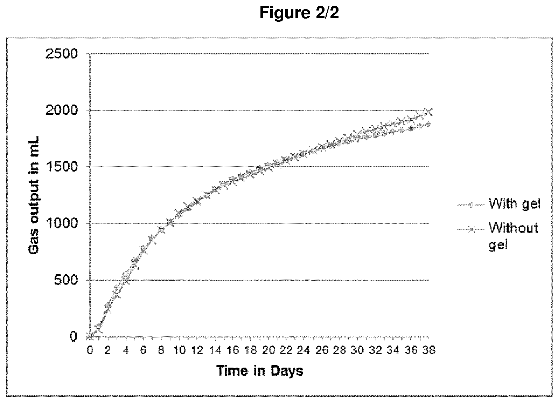

Figure 2 shows the comparison of biogas production over 38 days from a sample containing digestate and polymeric gel and a sample containing digestate alone. This may show that in the polymeric gel is not acting as a feedstock for the microbes.

### Detailed Description of the Invention

In a first aspect of the present invention there is provided a method for the hydrolysis of biomass in a fermenter, such as an anaerobic digester, comprising the steps of
(i) combining a microbial composition, a suitable feedstock, and a polymeric material, to form a mixture; and then
(ii) digesting the mixture in the fermenter under suitable conditions to hydrolyse the biomass.

It has been shown herein that the first aspect of the present invention provides an improved method for the hydrolysis of biomass, for instance in the formation of biogas.

As used herein, the term "biomass" is one of the art and refers to organic material.

The term "hydrolysis of biomass" as used herein refers to the breakdown of biomass into its constituent parts. Constituent parts of biomass include carbohydrates, lignins, cellulose, sugars, amino acids, aldehydes, alcohols, carboxylic acids.

The term "fermenter" as used herein has its usual meaning within the art, and is a vessel where fermentation takes place. The term "fermentation" refers to the breakdown of organic material by microorganisms. Within the context of the first aspect of the invention a particularly envisioned fermenter is an anaerobic digester.

As used herein the term "fermentation" refers to the breakdown of organic material by microorganisms.

The term "anaerobic digestion" specifically refers to the process wherein microorganisms breakdown organic material in the absence of oxygen. Anaerobic digestion converts biomass to biogas through a series of biological processes including a hydrolytic stage, acidogenesis, acetogenesis and methanogenesis. Each process is performed by suitable microorganisms, generally bacteria and archaea. The hydrolytic stage, acidogenesis and acetogenesis involve the breakdown of organic compounds and methanogenesis involves the production of methane, carbon dioxide and water. As anaerobic digestion does not require the presence of oxygen this means that it may be performed in an oxygen-free environment. By "oxygen-free", we mean less than about 20%, less than about 10%, less than about 5%, less than about 4%, less than about 3%, less than about 2%, less than about 1% oxygen present.

According to the first aspect of the invention the method is for the production of biogas, sugar, alcohol or carboxylic acid such as volatile fatty acid.

In a preferred feature of the first aspect of the present invention there is provided a method for producing biogas in an anaerobic digester, comprising the steps of
(i) combining a microbial composition, a suitable feedstock, and a polymeric material, to form a mixture; and then
(ii) digesting the mixture in the anaerobic digester under suitable conditions to produce biogas.

This particular method may provide an improved method for the production of biogas.

As mentioned above, the term "biogas" refers to the gas, or mixtures of gases, produced from the breakdown of organic material or "biomass". The biomass is broken down, or "digested", by microorganisms. Biogas typically comprises methane, but may also contain carbon dioxide and small amounts of hydrogen and hydrogen sulphide.

As used herein, the term "polymeric material" refers to a compound made of repeating units or "monomers". The polymeric material is preferably organic. It may comprise synthetic or natural repeating units. The repeating units can be arranged in a linear, branched or cross-linked manner. The polymeric material may be a copolymer, wherein a "copolymer" refers to a polymer comprising two or more different monomer units that are polymerised in a process called copolymerisation. Since a copolymer comprises at least two different monomer units, copolymers can be classified based on how the monomer units are arranged to form a polymer chain. Those classifications include "alternating copolymers" (in which the monomers units repeat with an regular alternating pattern), "periodic copolymers" (in which the monomers units are arranged with a repeating sequence), "statistical copolymers" (in which the sequence of monomer units follows a statistical rule), "random copolymers" (in which the monomer units are attached in a random order), and "block copolymers" (in which two or more homopolymer subunits are linked).

The copolymers of the invention may be a block copolymer, alternating copolymer, periodic copolymer, statistical copolymer or random copolymer. It is preferred that the copolymers are random copolymers.

The polymeric material may be in any physical form, however, it may be preferable to be in a discontinuous form, such as a powder, granule or particle form. The polymeric material may be crystalline, non-crystalline (amorphous), or a combination thereof.

Within the scope of the invention the term "polymeric material" does not refer to any component part of the fermenter machinery and is not part of any component required for the standard working of a fermenter. For example the polymeric material does not refer to the vessel, the mixer or agitator or the pipes and connectors. The polymeric material of the present invention is a component which is added into the fermenter and may be wholly removed from the fermenter should this be necessary.

A particular feature of the first aspect of the invention is that the polymeric material is a water-swellable (swellable) polymer or a water-swollen (swollen) polymer. Water-swellable polymers are well known to those skilled in the art and may be classified as "super-absorbant" polymers. The polymeric material may be provided in a "dried state", i.e. a dehydrated state, but may be capable of forming a gel when mixed with water (i.e. a "hydrogel"). The polymeric material may be provided in a semi-hydrated state, wherein the polymeric material has been mixed with water. To form a semi-hydrated state the polymer may have been mixed with an amount of water up to about 5%, about 10%, about 25%, about 50%, about 75%, about 100%, about 200%, about 500% of the polymer weight.

In the first aspect of the invention it is preferred that the polymeric material is a polymeric gel or a polymeric material capable of forming a polymeric gel. The polymeric gel may be water-absorbent.

None limiting examples of polymeric material that is suitable for use in the present invention include polyvinyl alcohol, methacrylate-polystyrene, starch-acrylonitrile, polyalkylene oxides, sodium acrylamide gel, potassium acrylamide gel ammonium acrylamide gel polymers formed of monomers of (meth)acrylic acid and/or (meth)acrylamide. Any derivatives of the polymers listed are also suitable. It is preferred that the polymeric material is a copolymer of acrylamide and acrylate. More preferably it is a cross-linked copolymer of acrylamide and potassium acrylate, a cross-linked copolymer of acrylamide and sodium acrylate, or a cross-linked copolymer of acrylamide and ammonium acrylate. An example of a suitable copolymer of acrylamide and acrylate is AQUASORB™ 3005 S.

According to the first aspect of the present invention when the polymeric material is a swollen or swellable polymeric material, it may be swollen with water or unswollen prior to its combination with the microbial composition and suitable feedstock. The polymeric material can be swollen with water by up to about 2 times, about 5 times, about 10 times, about 50 times, about 100 times, about 200 times, about 400 times its weight. A solution used to swell the polymeric material can comprise water and other components including additives such as glucose, sucrose, galactose, lactose, glycerol, urea, ammonium salts, nitrates. In a preferred embodiment of the first aspect of the invention the polymeric material is swollen with a solution containing glucose and urea before the polymeric material is combined with the microbial composition and suitable feedstock.

The microbial composition in the first aspect of the invention is suitable for use in fermentation and/or anaerobic digestion. It may be advantageous for the microbial composition to comprise obligate anaerobes and/or facultative anaerobes. Preferably the microbial composition comprises
(i) hydrolytic bacteria;
(ii) acidogenic bacteria;
(iii) acetogenic bacteria; and/or
(iv) methanogenic archaea.

It is particularly preferred that the microbial composition comprises hydrolytic bacteria, acidogenic bacteria, acetogenic bacteria and methanogenic archaea.

During the hydrolytic stage organic material, such as proteins, carbohydrates and fats, are broken down into soluble monomers, such as amino acids, monosaccharides, polysaccharides and fatty acids. Hydrolysis is carried out by relative anaerobes. The hydrolytic bacteria may comprise bacteria selected from the genera *Streptococcus,* and *Enterobacterium.*

During acidogenesis, acidifying bacteria convert the hydrolysis products and water-soluble substances into short chain fatty acids, aldehydes, alcohols, carbon dioxide and hydrogen. Organic acids produced include formic acid, acetic acid, butyric acid and pentanoic acid. Alcohols produced include methanol and ethanol. Bacteria involved in acidogenesis include both facultative anaerobes and obligate anaerobes. The acidogenic bacteria may comprise bacteria selected from the genera *Pseudomonas*, *Bacillus*, *Clostridium*, *Micrococcus,* and *Flavobacterium.*

Some of the products formed during acidogenensis, such as acetic acid, carbon dioxide and hydrogen, can be used directly in methanogenesis. However, other products such as the alcohols and short chain fatty acids require further breakdown via acetogenesis to form acetic acid carbon dioxide and hydrogen. The acetogenic bacteria may comprise bacteria selected from the genera *Syntrophomonas,* and *Syntrophobacter.*

During methanogesis the carbon dioxide, acetic acid and hydrogen is converted into methane. This process is performed by methane producing archaea, also referred to as methanogens, which are obligate anaerobes. The methanogenic archaea may comprise archaea selected from the genera *Methanobacterium, Methanobrevibacter, Methanosarcina*, *Methanomicrobium*, *Methanoculleus*, and *Methanosphaera, Methanosaeta.* Preferably, the archaea are selected from *Methanomicrobiales, Methanosarcinales*, *Methanobrevibacter ruminantium, Methanobacterium formicicum*, *Methanobrevibacter ruminantium*, *Methanosarcina barkeri*, and *Methanomicrobium mobile.*

Anaerobic digestion provides a method of producing biogas from waste products and as such is an attractive environmentally friendly system for energy production. To produce biogas a suitable feedstock is required. The term "suitable feedstock" refers to organic material or biomass which can be broken down. None limiting examples of suitable feedstocks useful in the method of the present invention include silage, grass clippings, straw, maize, food waste, sewage, waste paper, animal waste, manure and slurry. Feedstocks which are particularly envisioned in the present method include cellulose and hemicellulose containing feedstocks, for example silage from plant material and plant waste, preferably green crop silage.

The feedstock can be supplemented with additives. These additives may improve microbial growth in the anaerobic digester. Additives which are suitable for use in the first aspect of the invention include glucose, sucrose, galactose, lactose, glycerol, urea, ammonium salts, and nitrates. The feedstock may be supplemented with glucose and urea.

Feedstocks high in cellulose and hemicellulose may be difficult to breakdown into their constituent parts. Hydrolysis of cellulosic material requires a variety of enzymes. These enzymes are present in anaerobic cellulose degrading bacteria. Therefore, in the first aspect of the present invention it is advantageous that the microbial composition comprises bacteria capable of anaerobic cellulose degradation. By including cellulose degrading bacteria in the anaerobic digestion process, feedstocks containing cellulose are more efficiently broken down. None limiting examples of cellulose degrading bacteria include *Fibrobacter succinogenes, Ruminococcus albus*, and *Ruminococcus flavefaciens.* Some cellulose degrading bacteria can only degrade the cellulose from the "cut" end of the cellulose containing material. However, *Fibrobacter succinogenes* is understood not to be restricted to the cut end when degrading cellulose. To improve the breakdown of cellulose-containing feedstock, it is preferred that the microbial composition comprises *Fibrobacter succinogenes.*

In the first aspect of the invention is it advantageous that the microbial composition comprises hydrolytic bacteria, acidogenic bacteria, acetogenic bacteria, methanogenic archaea and anaerobic cellulose degrading bacteria, preferably *Fibrobacter succinogenes.* As such, high cellulose or hemicellulose-containing feedstocks are suitable for use in the first aspect of the invention. Wherein "high cellulose" feedstocks refers to feedstocks containing up to about 10%, about 20%, about 30%, about 40%, about 50%, about 60%, about 70%, about 80%, about 90%, about 100% w/w cellulose and "high hemicellulose" feedstocks refers to feedstocks containing up to about 10%, about 20%, about 30%, about 40%, about 50%, about 60%, about 70%, about 80%, about 90%, about 100% w/w hemicellulose. Preferable high cellulose feedstocks and high hemicellulose feedstocks include arable silage, preferably straw silage, maize silage, grass silage, corn silage and rye silage.

According to the first aspect of the present invention, the microbial composition is combined with a suitable feedstock and a polymeric material. The microbial composition may be present in a number of forms. For instance, the microbial composition may be contained within cattle slurry, wherein the term "cattle slurry" refers to a mixture of cow manure and water. The microbial composition may be contained within organic material which has already undergone anaerobic digestion. Such material may be sourced from an anaerobic digester. It is preferred that the microbial composition is contained within cattle slurry.

The microbial composition, suitable feedstock and polymeric material are combined in step (i) of the present method to form a mixture. The skilled person will understand that these components may be combined in any order, for example the microbial composition, suitable feedstock and polymeric material may be combined concomitantly or sequentially. It is also envisioned that two of the components may be combined and incubated together prior to the addition of the third component. In particular, the polymeric material and microbial composition may be combined and incubated prior to the addition of the suitable feedstock. For instance, the polymeric material and microbial composition may be combined and incubated under suitable conditions for microbial growth for up to about 1 day, about 2 days, about 3 days, about 4 days, about 5 days, about 6 days, about 7 days, about 8 days, about 9 days, about 10 days prior to combining with the suitable feedstock.

It may be advantageous, and therefore especially preferred, that the polymeric material is combined with glucose and urea prior to step (i) of the present method. This is particularly beneficial if the polymeric material is a swellable polymer or a swollen polymer, as described above. In this case, it is preferred that the polymeric material is in a semi-hydrated state, as discussed above.

Suitable conditions for microbial growth are well known to those skilled in the art. As mentioned above, the polymeric material may be swollen with water and/or suitable additives prior to being combined with the microbial composition.

Anaerobic digesters can be arranged in a continuous or batch configuration. A continuous configuration involves the feedstock and/or other components being continuously added, or added in stages, during the anaerobic digestion process. A batch configuration involves the feedstock and other components being added in a single stage. The method according to the first aspect of the present invention is suitable for use in an anaerobic digester configured for either continuous or batch use.

The skilled person will understand that advantages of using an anaerobic digester configured for continuous use. Therefore, it is preferred that additional feedstock, microbial composition and/or polymeric material are added into the anaerobic digester during the digestion process. The additional feedstock, microbial composition and/or polymeric material may be added in any order for example sequentially or concomitantly, or may be combined prior to addition to the anaerobic digester.

As discussed above, the digestion process is performed in an anaerobic digester which provides suitable conditions for biomass to be broken down or digested and converted into biogas. Suitable conditions for anaerobic digestion include mesophilic and thermophilic conditions. These conditions are known to those skilled in the art.

Under thermophilic conditions the temperature in the anaerobic digester may be greater than about 40°C, more preferably from about 40°C to about 60°C, even more preferably from about 45°C to about 55°C, most preferably about 50°C. Under mesophilic conditions the temperature may be from about 25°C to about 40°C, preferably from about 31 °C to about 39°C, most preferably about 35°C. It is preferred that the invention is performed under mesophilic conditions. It is preferred that the invention is performed under normal atmospheric pressure.

The pH in an anaerobic digester has an effect on the efficiency of the digestion process as the microbes and enzymes may be pH sensitive. The method of the present invention may be performed at a pH of from about pH 5 to about pH 8.5, preferably from about pH 6.5 to about pH 7.5, even more preferably at about pH 7. The skilled person will know how to determine an optimal pH at which to perform the anaerobic digestion process.

Biogas produced from the present method has various downstream uses. The biogas may be predominantly methane (i.e. approximately 60%) and carbon dioxide (i.e. approximately 40%) with trace amounts of hydrogen and hydrogen sulphide. In this form the biogas may be used in gas stoves. However, it is sub-optimal for use as a fuel, and may require special corrosion protected machinery due to the hydrogen sulphide. Therefore, the biogas may be to be further refined to form biomethane. This process involves separating, or extracting, the biomethane from the remaining components of biogas, such as carbon dioxide, hydrogen and hydrogen sulphide.

In a second aspect of the invention there is provided a method for forming biomethane comprising the steps of
a) forming biogas by the method according to the first aspect of the invention; and
b) extracting biomethane from the biogas formed in step a).

The biomethane may be extracted from the biogas formed by step a) by reducing the concentration of carbon dioxide, hydrogen and hydrogen sulphide. These "refinement" processes produce a gas with a higher content of biomethane. Methods to refine the biogas are well known in the art and the skilled person would be able to determine the most suitable method. None limiting examples of suitable methods include water absorption methods, polyethylene glycol scrubbing methods, carbon molecular sieve methods and membrane separation methods.

In a third aspect of the invention there is provided a kit for use in the method according to the first aspect of the invention. The kit comprises a polymeric material and instructions for its use in the method. The polymeric material may be as described in relation to the first aspect of the invention. In particular, polymeric material may be provided as an unswollen polymeric material or as a swollen polymeric material. It may be advantageous that the kit comprises an unswollen polymeric material. It may be advantageous that the kit comprises semi-hydrated polymeric material. Additional components may also be provided as part of the kit. Such components include additives, such as sugars and nitrogen containing compounds. These additional components may help to provide a suitable environment for microbial growth. None limiting examples of additives suitable for use in the kit include glucose, sucrose, galactose, lactose, glycerol, urea, ammonium salts, and nitrates. The additional components may be provided in separate containers or as a mixture with a polymeric material. The additional components can be provided as a mixture with the unswollen polymeric material or as a mixture with the swollen polymeric material or as a mixture with the semi-hydrated polymer. It is preferred that the kit comprises a polymeric material, glucose, urea and instructions for use in the method.

The kit may also comprise a microbial composition suitable for use in anaerobic digestion. The microbial composition may be as described in relation to the first aspect of the invention. The microbial composition may be provided as a microbial culture which can be combined with the polymeric material. A "microbial culture" refers to a microbial composition wherein the microbes are capable of reproducing. The culture may be contained within cattle slurry or digestate taken from an anaerobic digester or within appropriate microbial media. The microbial composition may be provided as a glycerol stock which can be stored at a temperature of from about -20°C to about -80°C. The preparation and use of glycerol stocks of microorganisms are well known to those skilled in the art and these stocks provide a convenient method for storage and transport of microbial compositions wherein the composition will remain viable. The microbial composition may require culturing prior to use in the kit. The term "culturing" refers to growth of the microorganisms under appropriate conditions for the microorganisms to multiply. Appropriate culturing conditions will be known to those skilled in the art.

The kit may therefore comprise a polymeric material, a microbial composition suitable for use in anaerobic digestion, and instructions for use in the method according to the first aspect of the invention. The polymeric material and the microbial composition may be provided in separate containers, or as a mixture. The mixture of polymeric material and the microbial composition may be prepared by culturing the microbial composition with the polymeric material under appropriate conditions. It may be advantageous that the microbial composition is provided as a mixture with the polymeric material in a semi-hydrated state.

The kit may comprise a suitable feedstock, such as a feedstock as described in relation to the first aspect of the invention. The kit may comprise a polymeric material, a microbial composition suitable for use in anaerobic digestion, a suitable feedstock and instructions for use according to the first aspect of the invention. The suitable feedstock may comprise silage, grass clippings, straw, maize, food waste, sewage, waste paper, animal waste, manure (such as solid manure) or slurry. The suitable feedstock may further comprise additives such as glucose, sucrose, galactose, lactose, glycerol, urea, ammonium salts, nitrates.

In a fourth aspect of the invention there is provided the use of a microbial composition and a polymeric material as defined for the first aspect of the invention, in the production of biogas by anaerobic digestion in an anaerobic digester, wherein the microbial composition is suitable for use in anaerobic digestion.

The microbial composition and polymeric material of the fourth aspect of the invention may be as defined for the first aspect of the invention. The polymeric material may be a water-swellable polymer. Preferably the polymeric material is a copolymer of acrylamide and acrylate. More preferably it is a cross-linked copolymer of acrylamide and potassium acrylate, or a cross-linked copolymer of acrylamide and sodium acrylate, or a cross-linked copolymer of acrylamide and ammonium acrylate. The polymer may be provided in an unswollen form or in a swollen form.

The microbial composition is preferably suitable for use in anaerobic digestion. Specific examples of microoragnisms suitable for use in anaerobic digestion include hydrolytic bacteria, acidogenic bacteria, acetogenic bacteria, methanogenic archaea. In a preferred embodiment of the fourth aspect of the invention the microbial composition comprises *Fibrobacter succinogenes.* In a more preferred embodiment, the microbial composition comprises hydrolytic bacteria, acidogenic bacteria, acetogenic bacteria, methanogenic archaea and *Fibrobacter succinogenes.*

### Examples

The following examples of the invention should not be construed as limiting in any way.

### Example 1 - Biogas output from anaerobic digestion

Experiments were performed to determine the amount of biogas produced from anaerobic digestion in the presence of a polymeric material. The polymeric material used throughout the experiments was AQUASORB™ referred to as "gel".

9g of volatile solids of feedstock were added to the vessels in two stages, as below. Additionally a total of 30g of gel was added in two stages. The vessels were inoculated with digestate obtained from another anaerobic digester. The digestate comprises the microbial composition.

In stage one, a total of 3 g of volatile solids of feedstock were added with 20g of gel and left for 9 days. The volatile solids were provided in the form of grass silage. The volatile solid content of the grass silage was determined by heating a known amount of sample at 550°C in a Thermolyne muffle furnace until a steady weight was reached. This gave a measure of the organic portion of the sample, may be the material potentially available to the microbes.

In stage two, the remaining 6g of feedstock volatile solids were added with an additional 10g of gel. This was left to run for a further 28 days. The process was performed under standard mesophilic conditions.

The results are shown in Figure 1, which illustrates the gas output comparing the feedstock and digestate both in the presence and absence of the polymeric material. The results show that the polymeric material has a beneficial effect on gas output in stage one - up to day nine. The most dramatic increase is seen after day nine, which is the point of the second addition of feedstock. There is a rapid rise in the gas output from day nine up to day 20.

In the sample without the polymeric material, from day nine onwards there is a steady increase in gas output which is slower than with the polymeric material up to day 20. Over the course of the experiment the sample with polymeric material achieves a higher rate and a greater overall production of gas. By day 19 of the experiment the sample with polymeric material had reached a higher overall gas volume than the sample without polymeric material reached by the end of the experiment (day 38).

The anaerobic digestion process was performed over a total of 38 days. The process was performed both with and without the polymeric material. The amount of gas produced per tonne of feedstock is summarised in Table 1 shown below.

Table 1 illustrates the total gas output for the feedstock with and without polymeric material added. As can be seen, there is a significant increase in gas output at the end of the experiment. The difference between the two samples represents a 33.6% increase in gas production in this experiment.

**Table 1**

| **Sample** | **Gas output M³**/**Tonne** |
|---|---|
| Feedstock with Gel | 119.25 |
| Feedstock without Gel | 89.25 |

### Example 2 - Polymeric material is not a feedstock

Control experiments were performed which contained digestate both with and without gel i.e. no cellulosic material is present. These were used to test the effect of the gel alone on the production of biogas. Samples were analysed in triplicate. The results of the control experiment of digestate with and without the polymeric material are shown in Figure 2. The graph shows that when there is no cellulosic material present the polymeric material has no effect on the production of biogas. Clearly the polymeric material is not acting as a feedstock for the microbes.

From these results the polymeric material causes an increase (approximately 34% as shown in table 1) in the production of biogas from anaerobic digestion. The control shows that polymeric material does not contribute to gas production when no cellulosic material is present. As such, the polymeric material may provide a more suitable environment for the microbial composition to utilise feedstock.

## Claims

1. A method for the hydrolysis of biomass in a fermenter, such as an anaerobic digester, comprising the steps of
(i) combining a microbial composition, a suitable feedstock, and a polymeric material, to form a mixture; and then
(ii) digesting the mixture in the fermenter under suitable conditions to hydrolyse the biomass.

2. The method according to claim 1, wherein the method is for the production of biogas, sugar, alcohol and/or carboxylic acid, such as volatile fatty acid.

3. The method according to any preceding claim, wherein the method is for the production of biogas in an anaerobic digester, comprising the steps of
(i) combining a microbial composition, a suitable feedstock, and a polymeric material, to form a mixture; and then
(ii) digesting the mixture in the anaerobic digester under suitable conditions to produce biogas.

4. The method according to any preceding claim, wherein the polymeric material is a polymeric gel.

5. The method according to any preceding claim, wherein the polymeric material is selected from the group consisting of polyvinyl alcohol, methacrylate-polystyrene, starch-acrylonitrile, polyalkylene oxides, sodium acrylamide gel, potassium acrylamide gel, ammonium acrylamide gel, and a polymer of monomers of (meth)acrylic acid and/or (meth)acrylamide, or a derivative thereof, or a combination thereof, preferably wherein the polymeric material is copolymer of acrylamide and acrylate.

6. The method according to any preceding claim, wherein the microbial composition is suitable for use in fermentation and/or anaerobic digestion.

7. The method according to any preceding claim, wherein the microbial composition is contained within cattle slurry.

8. The method according to any preceding claim, wherein the microbial composition comprises
(i) hydrolytic bacteria;
(ii) acidogenic bacteria;
(iii) acetogenic bacteria; and/or
(iv) methanogenic archaea.

9. The method of any preceding claim, wherein the microbial composition comprises bacteria capable of anaerobic cellulose degradation, preferably *Fibrobacter succinogenes.*

10. The method according to any preceding claim, wherein the suitable feedstock comprises
a) cellulosic material; and/or
b) hemicellulosic material; and/or
c) sugar and urea.

11. The method according to any preceding claim, wherein in step (ii), additional microbial composition, feedstock and/or polymeric material are added to the fermenter.

12. The method according to any preceding claim, wherein the digestion is performed under mesophilic conditions.

13. A method for forming biomethane comprising the steps of
a) forming biogas by a method as defined in any of claims 1 to 12; and
b) extracting biomethane from the biogas formed in step a).

14. A kit for use in the method of any of claims 1 to 12, wherein the kit comprises a polymeric material and instructions for its use in the method.

15. The use of a microbial composition as defined in any one of claims 1 to 12, and a polymeric material as defined in any one of claims 1 to 12, in the production of biogas by anaerobic digestion in an anaerobic digester, wherein the microbial composition is suitable for use in anaerobic digestion.
